# EUROPEAN PATENT APPLICATION

(11) **EP 3 708 081 A1**
(43) Date of publication of application: **16.09.2020**
(21) Application number: 18806573.4
(22) Date of filing: 24.07.2018
(51) Int. Cl.: A61B 5/151

(54) **CONTACT PRESSURE-TYPE DISPOSABLE BLOOD COLLECTION DEVICE PROVIDED WITH SPRING GUIDE STRUCTURE**

(71) Applicant: Sterilance Medical (Suzhou) Inc., Jiangsu 215133 (CN)
(72) Inventor: SHI, Guoping, Suzhou Jiangsu 215133 (CN)
(74) Representative: Betten & Resch
(86) International application number: PCT/CN2018/096909
(87) International publication number: WO 2018/214993

(57) **Abstract**

A pressing type disposable safety lancet with spring pilot structure consists of a shell, a lancet core, a spring and a trigger; wherein: the spring is provided with a pilot structure at the outside to make the elastic force of the spring act along the ejection direction of the lancet core; the locking mechanism is formed by the cooperation of the elastic clamp at the tail of the trigger and the lancet core; the unlocking mechanism is formed by the front of pilot structure and the end of the elastic clamp through the unlocking bevel; when it's pressed during the use, the trigger moves backwards in the shell, the front of the pilot structure forces the end of the elastic clamp to open under the action of the unlocking bevel, and the lancet core is fired for puncturing under the action of the spring after the hook unhooks from the locking critical point. The solution associates the spring pilot structure and the unlocking mechanism and skillfully uses one structure for two functions, thereby achieving the dual effect of piloting and unlocking and reducing the shaking of the needle tip, simplifying the structure and reducing the cost.

## Description

### TECHNICAL FIELD

The present invention relates to the medical lancet field, in particular to a pressing type disposable safety lancet (or lancet) with a built-in ejection mechanism and a spring pilot structure. The biggest characteristic of the safety lancet is that it can reduce the shaking of the needle tip and reduce the pain, making people feel no pain or minimal pain.

### BACKGROUND OF INVENTION

In the field of medical blood sampling, the disposable lancet is popular among medical personnel and patients because of its small size, safety use and convenient operation. It is currently widely used by various medical institutions and diabetics. The safety lancet has a built-in ejection mechanism and compact structure, and it's disposable as a whole to be safe and convenient, so it has great market development potential.

With the development of the market, the types and forms of medical disposable safety lancets are various. However, from the perspective of product design, the first condition for measuring the quality of a safety lancet is to consider the shooting and puncturing performance of the lancet, wherein the shaking amplitude of the needle tip during it's punctured into the human skin is one of the important indicators, as it's not only related to the blood collection effect of the needle tip, but more importantly, it directly affects the pain of the puncturing. Particularly in today's society, the safety and user experience of the safety lancet in use has risen to a major factor to be considered in the product design. As for preventing the needle tip of the safety lancet from shaking, there are many factors affecting the shaking of the needle tip theoretically, such as the shooting guided structure of lancet core, locking mechanism of lancet core, triggering structure of lancet core and spring performance, etc. However, the most important consideration in the prior designs is to guide the shooting of the lancet core, that is, the guiding slot is provided to guide the shooting of the lancet core to reduce the shaking of the needle tip during the puncturing, while the other factors are neglected.

On the other hand, as a popular and mature safety lancet, it shall not only have good puncturing performance and reliability, but also have the characteristics of simple structure easy for industrial mass production and low manufacturing cost. Therefore, it's the future development direction of medical safety lancets and also the double problem faced by the technicians in this field to consider how to reduce the shaking of needle tip, fully consider the user experience, simplify the structural design and reduce the manufacturing cost based on the prior art.

### DISCLOSURE OF THE INVENTION

In view of the above problems in the prior art, the present invention provides a pressing type disposable safety lancet with spring pilot structure, with the first purpose of solving the problem of shaking during the puncturing of the above lancet and with the second purpose of simplifying the structure and reducing the cost.

In order to achieve the above purpose, the technical solution adopted by the present invention is: a pressing type disposable safety lancet with spring pilot structure consisting of a shell, a lancet core, a spring and a trigger.

The shell forms an ejecting cavity, the lancet core is positioned and locked in the ejecting cavity by the locking mechanism, the spring acts on the lancet core with its front end and an ejection guiding structure is provided between the lancet core and the shell or the trigger.

The trigger head extends out of the front end of the shell and works as the triggering end for blood collection, the trigger body is sliding connected in the shell and the tail end of the trigger cooperates with the locking mechanism to form an unlocking mechanism to form a head pressing type firing structure.

Wherein: As for the spring is provided with a pilot structure for piloting, the pilot structure is located in the shell and arranged on the outer circumference of the spring; the pilot structure comprises at least three pilot strips, or at least two pilot slices, or a pilot tube, and the pilot tube or all pilot slices or all pilot strips are arranged on the outer circumference of the spring to form a pilot channel confining the spring, and the spring is located in the pilot channel, so that the elastic force of the spring acts on the ejection direction of the lancet core, and the rear end of the spring acts on the pilot structure or the end cover of the shell.

The locking mechanism is formed by the cooperation of the trigger and the lancet core, wherein the trigger is provided with at least an elastic arm at the tail end and the elastic arm is provided with a hook or end face to lock the lancet core, and all elastic arms form an elastic clamp together or the elastic arm forms an elastic clamp with one of the inner wall of the shell, inner wall of the trigger and inner wall of the pilot structure, and the lancet core is clamped, positioned and locked in the elastic clamp to form the locking mechanism.

The unlocking mechanism is formed by the cooperation of the pilot structure and the trigger, wherein, the front of the pilot structure is set to face the back of the elastic arm, and at least one of the front of the pilot structure and the back of the elastic arm is set with an unlocking bevel.

Under the use state, when the trigger head is pressed, the trigger moves backwards in the shell, the front of the pilot structure hits the back of the elastic arm and forces the end of the elastic arm to open under the action of the unlocking bevel, and the lancet core is fired for puncturing under the action of the spring after the hook or end face on the elastic arm unhooks from the locking critical point.

The above described technical solution and the change are explained as follows:
1. In above described technical solution, the "shell", "lancet core", "spring" and "trigger" are the basic structures of the head pressing type disposable lancet, and the basic function and function are the prior art.
2. In above described technical solution, the "front" in the "front end" and "front part" means the pointing direction of the needle tip in the lancet. The "rear" in said "rear end" and "rear part" means the direction opposite to the "front". The "trigger head" means the end of the trigger facing the front, while the "trigger tail" means the end of the trigger facing the rear.
3. In above described technical solution, the "locking mechanism" means a structure of lancet used to lock the lancet core under the state before firing. The "unlocking mechanism" means a structure of lancet used to trigger the ejection of lancet core under the use state.
4. In above described technical solution, the "bevel" in the "unlocking bevel" includes the flat face, cambered face or tapered face, but it's always set obliquely in relative to the axis of lancing device to constitute a bevel. In other words, the bevel has four types: the bevel in the form of flat face, the bevel in the form of concave face (the bevel comprises several straight lines in the oblique direction, and the concave face or convex face are formed in the vertical direction, but it's substantially bevel), the bevel in the form of tapered face and the bevel in the form of cambered face (the bevel comprises at least one concave arc or/and convex arc in the oblique direction, but it's substantially bevel). In the present invention, any type is applicable and it could be determined according to the practical situation. Wherein, the first type is the bevel in the form of flat face; the second type is the bevel in the form of concave face; the third type is the bevel in the form of tapered face; and the fourth type is the bevel in the form of cambered face.
5. The pressing type disposable lancet of present invention consists of two structures of twist cap and cover cap. That is, the disposable lancet is usually designed with the protection cap, and the structure of protection cap could be the twist cap type or cover cap type. This doesn't affect the realization of the purpose of present invention.

The design principle and concept of the present invention are: in order to reduce the shaking of the needle tip, simplify structure, and reduce cost, the present invention adopts the following technical measures: Firstly, a pilot structure for confining the spring is arranged outside the spring, and the pilot structure can ensure the pilot effect during the assembly, firing and retraction state of the spring; Secondly, the pilot structure particularly comprises at least three pilot strips, or at least two pilot slices, or a pilot tube, and these structures advantageously simplify the structure and reduce the cost; Thirdly, the locking mechanism is designed between the trigger and the lancet core, that is, an elastic clamp is provided at the trigger tail and the elastic clamp is used to clamp and lock the lancet core; Fourthly, the movement and cooperation of the front of the pilot structure and the end of the elastic clamp is skillfully used to achieve the unlocking of the lancet core with the help of the unlocking bevel, that is, the spring pilot structure and the unlocking mechanism are skillfully associated to use one structure for two functions, thereby achieving the dual effect of piloting and unlocking.

Due to the application of the above described technical solution, the present invention has the following advantages and effect in comparison with the prior art:
1. The present invention has a significant effect of reducing the shaking of needle tip and reducing the puncturing pain after the pilot structure is added outside of the spring. According to the research finding, besides the deflection of needle tip among the factors causing the pain, the main factor is the shaking (or wobbling) of needle tip in the firing path. In the existing designs, in order to reduce the shaking of needle tip during the puncturing, only the guiding slot was provided to guide the shooting of the lancet core, while the influence of skew exertion of the spring was neglected. The spring is the drive component to fire the lancet core in the lancet, and if the spring is distorted under the assembly and compression state, it will cause the skew exertion of the spring during the ejection, which directly affect the shaking amplitude and verticality of needle tip during the puncturing. There are two main factors causing the skew exertion of the spring: the first one is that the spring is laterally swelled and twisted in the middle under the assembly and compression state of the spring; the second one is that the spring is not assembled and positioned accurately. The present invention corrects the assembly posture and stress of the spring through the pilot structure to ensure that the spring is always in the piloted state during the firing, retraction and resonance processes.
2. The invention can greatly improve the uniformity of the spring assembly posture and the stress after the pilot structure is added outside of the spring.
3. The present invention skillfully uses the movement and cooperation of the front of the pilot structure and the end of the elastic clamp to achieve the unlocking of the lancet core with the help of the unlocking bevel, that is, the spring pilot structure and the unlocking mechanism are skillfully associated to skillfully use one structure for two functions, thereby achieving the dual effect of piloting and unlocking.
4. The present invention uses the pilot structure and the cooperation of the front of pilot structure and the end of the elastic clamp to form an unlocking mechanism, which reduces the shaking of the needle tip, simplifies the structure and reduces the cost.
5. The present invention overcomes the disadvantage of only considering the sliding guide of the lancet core, while neglecting the effect of the spring as a drive component causing the shaking of the needle tip in the prior art. The pilot structure could reduce the shaking of the needle tip and reducing the pain to make people feel no pain or minimal pain, therefore the present invention has the substantive feature and significant progress.

### BRIEF DESCRIPTION OF THE DRAWINGS

Figure 1 shows the perspective view of embodiment 1 of present invention;
Figure 2 shows the exploded view of embodiment 1 of present invention;
Figure 3 shows the perspective view of the lancet core and twist cap of the embodiment 1 of present invention;
Figure 4 shows a local enlarged view of Figure 3;
Figure 5 shows the perspective view of the trigger of the embodiment 1 of present invention;
Figure 6 shows the exploded section view of the shell of embodiment 1 of present invention;
Figure 7 shows the exploded section view of embodiment 1 of present invention;
Figure 8 shows the section view of embodiment 1 of present invention in the initial assembly state;
Figure 9 shows the section view of embodiment 1 of present invention pressing the trigger after removal of the needle cap;
Figure 10 shows the section view of embodiment 1 of present invention in the state of unhooking the lancet core;
Figure 11 shows the perspective view of the first variant of the pilot structure of embodiment 1 of present invention;
Figure 12 shows the perspective view of the second variant of the pilot structure of embodiment 1 of present invention;
Figure 13 shows the exploded view of the variant with the end cap and shell as discrete component of embodiment 1 of present invention;
Figure 14 is perspective view of the discrete component end cap with pilot slice in the embodiment 1 of present invention;
Figure 15 is perspective view of the discrete component end cap with pilot tube in the embodiment 1 of present invention;
Figure 16 shows the section view of embodiment 1 of present invention in the initial assembly state omitting the shoulder;
Figure 17 shows the section view of embodiment 1 of present invention in the post firing state omitting the shoulder;
Figure 18 shows the perspective view of embodiment 2 of present invention;
Figure 19 shows the exploded view of embodiment 2 of present invention;
Figure 20 shows the section view of embodiment 2 of present invention in the initial assembly state;
Figure 21 shows the section view of embodiment 2 of present invention pressing the trigger after removal of the needle cap;
Figure 22 shows the section view of embodiment 2 of present invention in the state before unhooking the lancet core;
Figure 23 shows the perspective view of the shell with pilot strip of embodiment 2 of present invention;
Figure 24 shows the perspective view of the shell with pilot slice of embodiment 2 of present invention;
Figure 25 shows the perspective view of the shell with pilot tube of embodiment 2 of present invention;
Figure 26 shows the section view of embodiment 3 of present invention in the state before unhooking the lancet core;
Figure 27 shows the section view of embodiment 4 of present invention in the state before unhooking the lancet core;
Figure 28 shows the section view of embodiment 5 of present invention in the state before unhooking the lancet core;
Figure 29 shows the perspective view of the lancet with adjusting head structure of embodiment 6 of present invention;
Figure 30 shows the exploded view of the lancet with adjusting head structure of embodiment 6 of present invention;

The number description of above described drawings is as follows:
1. Shell; 2. Lancet core; 3. Spring; 4. Trigger; 5. Elastic arm; 6. Hook; 7. Retractile neck; 8. Clamping face; 9. Needle outlet hole; 10. Unlocking bevel; 11. Twist cap; 12. Hook; 13. Shoulder; 14. Inner end face; 15. Twisting part; 16. Protection rod; 17. Pilot strip; 18. Pilot slice; 19. Pilot tube; 20. Adjusting cap; 21. End cap; 22. Initial hook; 23. Triggering hook; 24. Hook face; 25. End face.

### SPECIFIC EMBODIMENT

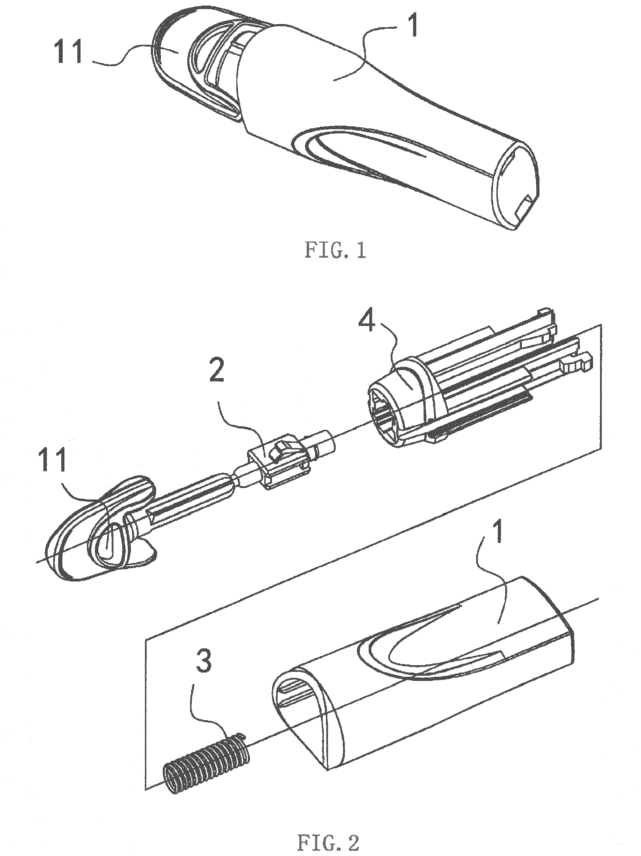

With reference to the accompanying drawings and embodiment, the present invention will be described in detail.
Embodiment 1: A pressing type disposable safety lancet with spring pilot structure (twist cap type) As shown in Fig. 1-10, the disposal safety lancet consists of a shell 1, a twist cap 11, a lancet core 2, a spring 3 and a trigger 4 (see Fig. 1 and 2).

The shell 1 forms an ejecting cavity, the lancet core 2 is positioned and locked in the ejecting cavity by a locking mechanism, the spring 3 presses the lancet core 2 with its front end and a ejection guiding structure is provided between the lancet core 2 and the trigger 4 (not shown in the figure), and the ejection guiding structure is particularly a sliding channel used for guiding.

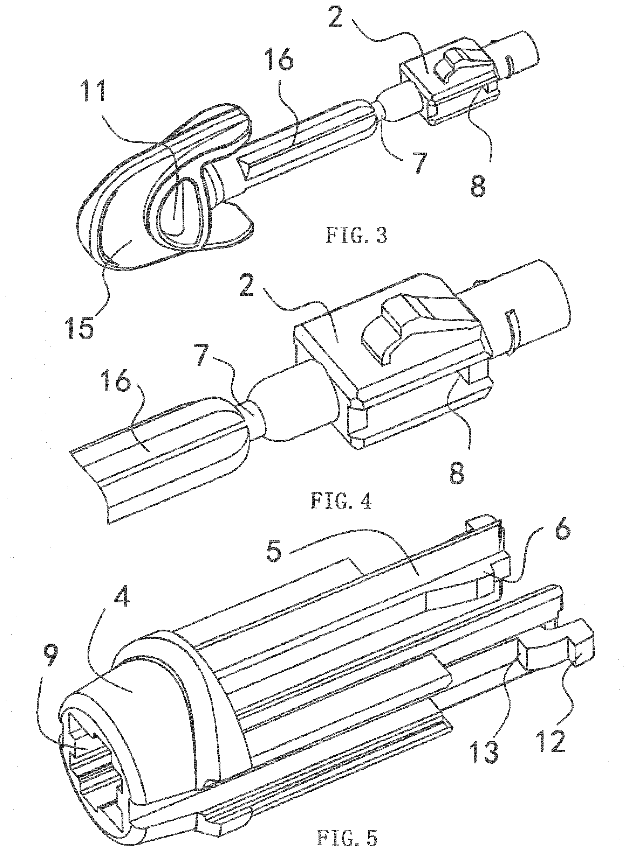

The twist cap is positioned in the front of the lancet core 2 and it's connected with the lancet core 2 to be an injection molding structure (as shown in Fig. 3 and Fig. 4), which could be deemed as a component. The twist cap is formed by the fixed connection of a twisting part 15 and a protection rod 16 (see Fig. 3) and a tetractile neck 7 that can be twisted off is provided between the protection rod 16 and the lancet core 2. The lancet core 2 is provided with a steel needle inside and the protection rod 16 is covered on the needle tip of the steel needle to protect the steel needle. The needle tip of the steel needle exposes from the front end of the lancet core 2 after the twist cap is removed for puncturing and blood sampling. The trigger 4 is provided with a needle outlet hole 9 at its head part (see Fig. 5) and the maximum diameter of the twisting part 15 is larger than the maximum diameter of the needle outlet hole 9, and the protection rod 16 and the lancet core 2 can be inserted through for assembly from the front end of the needle outlet hole 9.

The head of the trigger 4 extends out of the front end of the shell 1 and acts as the triggering end for blood sampling, the body of the trigger 4 is sliding connected in the shell 1 and the tail of the trigger 4 cooperates with the locking mechanism to form the unlocking mechanism, thereby forming the head pressing type firing structure.

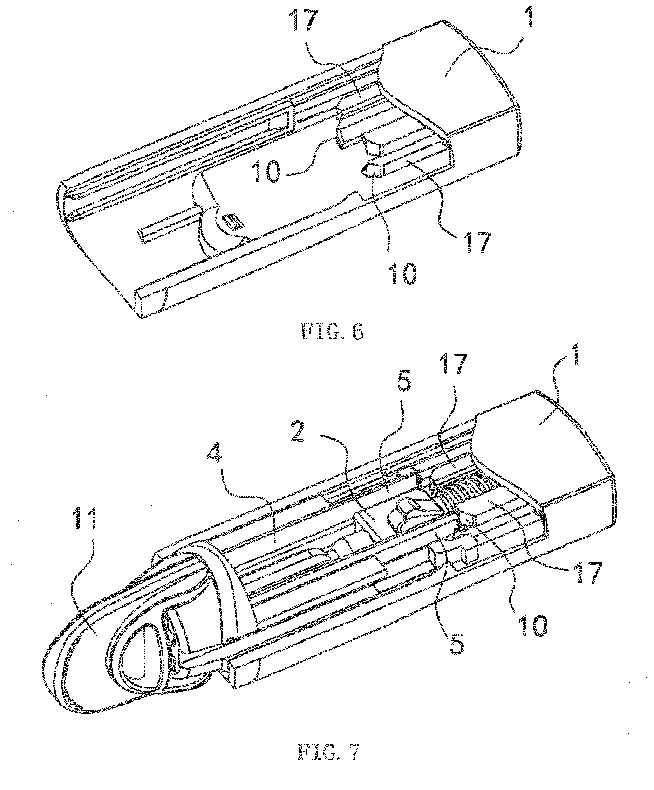

The present invention provides a pilot structure for a spring 3 for piloting and the pilot structure is positioned in the shell 1 and arranged on the outer circumference of the spring 3. The pilot structure comprises at least three pilot strips 17 and in this embodiment, the pilot structure actually comprises four pilot strips 17 (see Fig. 6), and five or six pilot strips are definitely possible, which is easily understood by those skilled in the art. All of the pilot strips 17 are arranged around the spring 3 circumferentially and form a pilot channel confining the spring 3, and the spring 3 is positioned in the pilot channel, so that the elastic force of the spring 3 act along the ejection direction of the lancet core 2. The pilot structure is the structure extending from the end cap of the shell 1 (see Fig. 6). The spring 3 acts on the end cap of the shell 1 at its rear end.

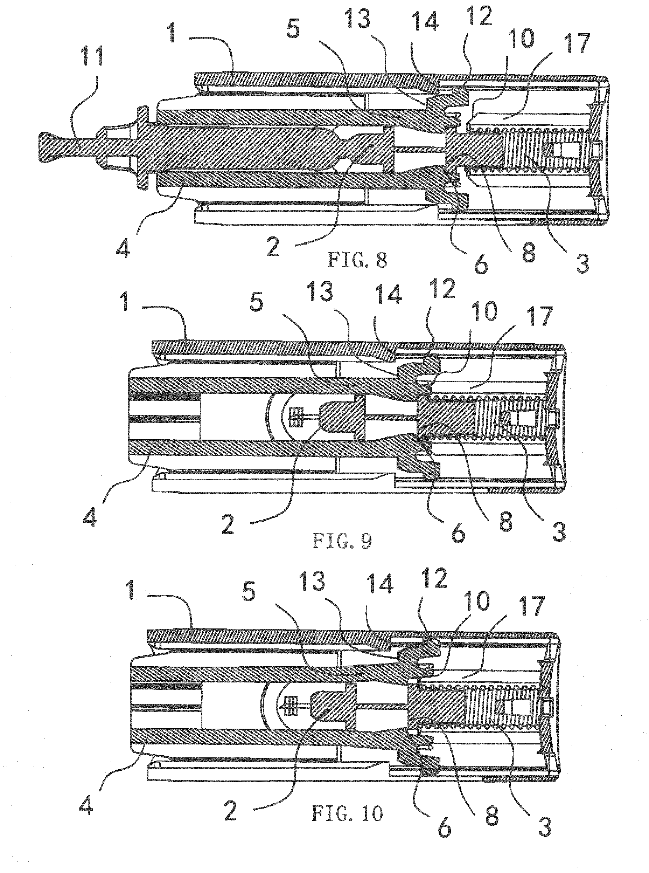

The locking mechanism is formed by the cooperation of the trigger 4 and the lancet core 2, wherein, the trigger 4 is provided with two elastic arms 5 at the tail (see Fig. 5), the elastic arm 5 is provided with a hook 6 to lock the lancet core 2, and the lancet core 2 is provided with a clamping face 8 relative to the hook 6 (see Fig. 3 and Fig. 4), and the clamping face 8 cooperates with the hook 6 to lock the lancet core 2 (see Fig. 8), thereby forming the locking mechanism.

The unlocking mechanism is formed by the cooperation of the pilot structure and the trigger 4, wherein, the front of the pilot structure is set to face the back of the elastic arm 5, and at least one of the front of the pilot structure and the back of the elastic arm 5 is set with an unlocking bevel 10. In this embodiment, the unlocking bevel 10 is provided at the front of the pilot structure, that is, the front end of the pilot strip 17 (see Figs. 6-10). Of course, it is also possible to provide the unlocking bevel 10 at the rear end of the elastic arm 5, or to provide the unlocking bevel 10 both on the front end of the pilot structure and rear end of the elastic arm 5.

The trigger 4 is provided with a hook 12 and a shoulder at the side (see Fig. 5) and the shell 1 is provided with an inner end face 14 on the inner wall relative to the hook 12 and the shoulder 13 (see Fig. 8). In the assembly state before firing, the hook 12 on the trigger 4 cooperates with the inner end face 14 to form a first limit to prevent the forward movement of the trigger 4 relative to the shell 1.

The action processes during the use of this embodiment are as follows:

### (1) Initial assembly state

As shown in Fig. 7 and Fig. 8, the whole lancet comprises a shell 1, a twist cap 11, a lancet core 2, a spring 3 and a trigger 4, wherein, the spring 3 is in the pre-pressed state and in the assembly state before firing, all elastic arms 5 form an elastic clamp together, and the lancet core 2 is clamped, positioned and locked in the elastic clamp through the clamping face 8, wherein, the hook 6 on the elastic arm 5 cooperates with the clamping face 8 on the lancet core 2 to make the lancet core 2 in the locking state. The rear end of the elastic arm 5 is arranged at the front of the unlocking bevel 10 (the front end of the pilot strip 17). The hook 12 on the trigger 4 cooperates with the inner end face 14 to form a first limit to prevent the forward movement of the trigger 4 relative to the shell 1.

### (2) State of pressing the trigger after the removal of the needle cap

As shown in Fig. 9, hold the lancet shell 1 with one hand and use the other hand to twist and remove the twist cap 11. Then, hold the shell 1 to align the lancet head with the blood sampling position for pressing and when the trigger 4 head is pressed, the trigger 4 moves backwards relative to the shell 1.

### (3) Lancet core unhooking state

As shown in Fig. 10, when the trigger continuously moves backwards in the shell, the front of the pilot structure (i.e. pilot strip 17) hits the back of the elastic arm 5 and forces the end of the elastic arm 5 to open under the action of the unlocking bevel 10, and the lancet core 2 is fired for puncturing under the action of the spring 3 after the hook 6 on the elastic arm 5 unhooks from the locking critical point.

### (4) Post firing state

In the post firing state (no figure), the shoulder 13 on the trigger 4 cooperates with the inner end face 14 to form a second limit to prevent the trigger 4 from returning relative to the shell 1, so that the trigger 4 head is in the flush or retracted state relative to the shell 1 head.

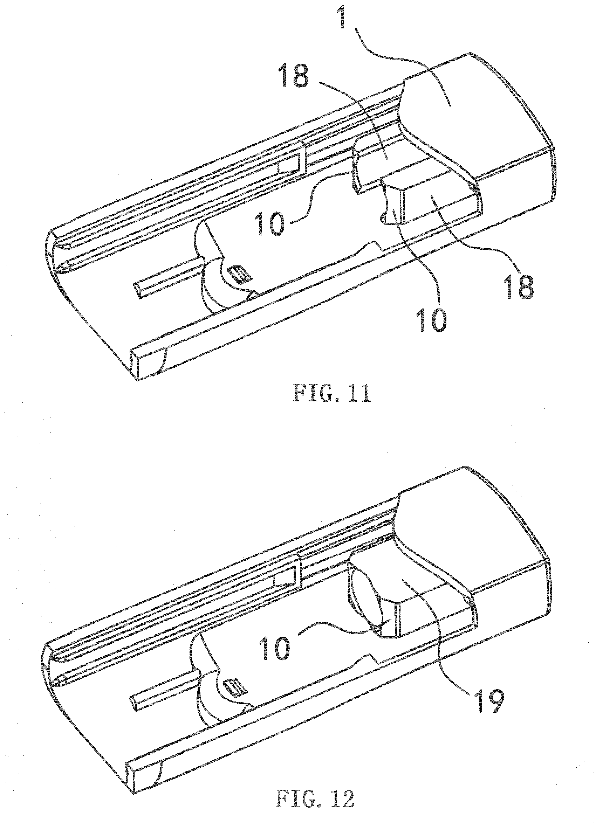

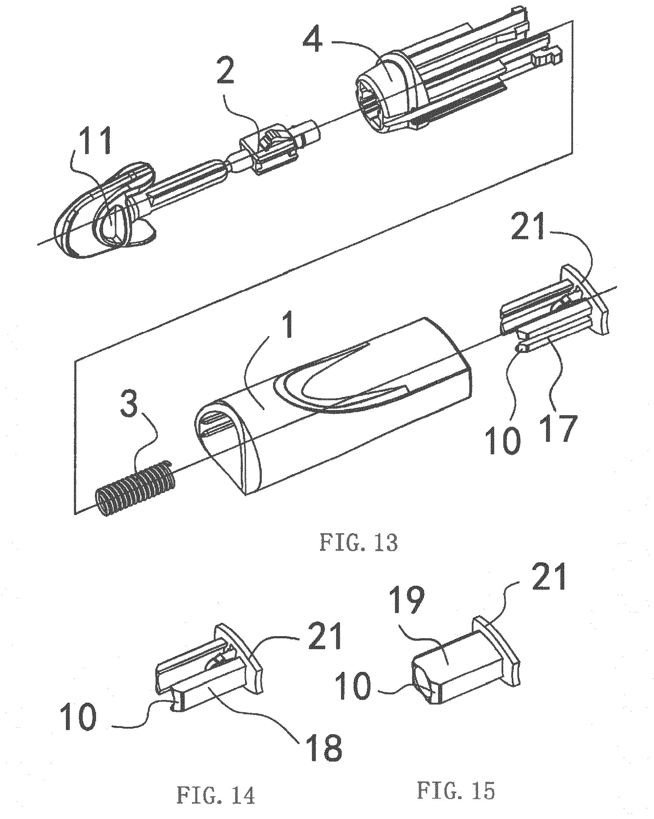

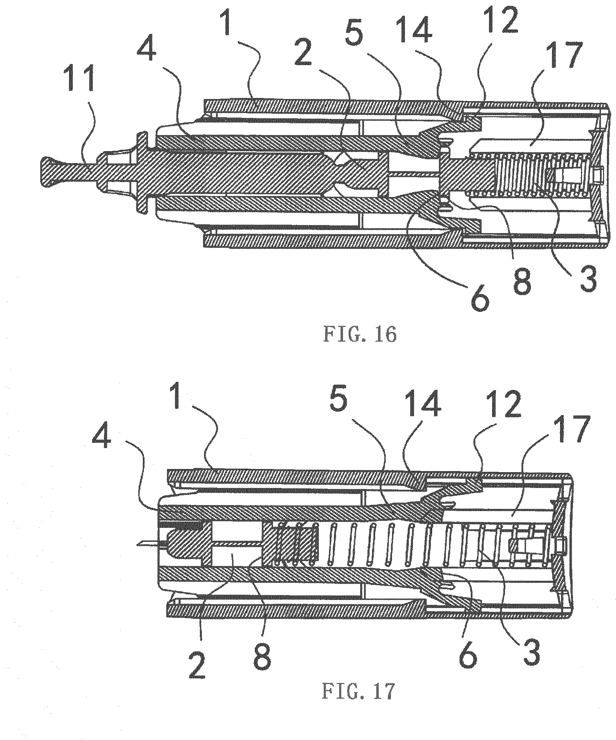

The following is a description of possible variants of the Embodiment 1.
1. Although the above embodiment 1 provides a twist cap type head pressing type disposable lancet, the present invention is not limited thereto, and is also applicable to a cover cap type head pressing type disposable lancet. The twist cap and cover cap are two forms of the protection caps of the lancet, but these two forms are not related to the innovation of the present invention, and it will be understood and recognized by those skilled in the art.
2. In the above embodiment 1, the trigger 4 is provided with two elastic arms 5 at the tail (see Fig. 5) and two elastic arms form an elastic clamp used to clamp and lock the lancet core 2. However, the present invention is not limited thereto, and the elastic arms 5 forming the elastic clamp may be two, three, four, five, six, etc. in terms of the number of the elastic arms. It will be understood and recognized by those skilled in the art.
3. In the above embodiment 1, as can be seen from the Fig. 5 that the elastic arm 5 is the straight arm structure. But the present invention is not limited thereto, and the elastic arm 5 may also be a curved arm structure or a folded arm structure. It will be understood and recognized by those skilled in the art based on the embodiment 1.
4. In the above embodiment 1, the spring 3 is an individual metal part, and the spring 3 can also be designed as a plastic spring and integrated with the lancet core 2. This is the change that could be easily understood by those skilled in the art.
5. In the above embodiment 1, an ejection guiding structure (sliding channel) is provided between the lancet core 2 and the trigger 4. But the present invention is not limited thereto, and the ejection guiding structure (sliding channel) can also be provided between the lancet core 2 and the shell 1. It will be understood and recognized by those skilled in the art.
6. In the above embodiment 1, the pilot structure comprises four pilot strips (see Fig. 6). But the present invention is not limited thereto, and the pilot structure can also comprise at least two pilot slices 18 (see Fig. 11) or comprise a pilot tube 19 (see Fig. 12). It will be easy to be understood and recognized by those skilled in the art based on the embodiment 1.
7. In the above embodiment 1, the pilot structure is the structure extending from the end cap of the shell 1. But the present invention is not limited thereto, and the pilot structure can also be the structure extending from the inner wall of the shell 1 and it can also be the structure extending from the end cap and inner wall of the shell 1 at the same time. It will be understood and recognized by those skilled in the art.
8. In the above embodiment 1, the spring 3 acts on the end cap of the shell 1 at its rear end. But the present invention is not limited thereto, and the spring 3 can also act on the pilot structure at its rear end. It will be understood and recognized by those skilled in the art.
9. In the above embodiment 1, the end cap is part of the shell 1 and the end cap and the shell 1 are integrally molded and deemed as a component. But the present invention is not limited thereto, and the end cap and the shell can be processed and molded as two components to facilitate the processing. It will be understood and recognized by those skilled in the art based on the embodiment 1. In Fig. 13, the end cap 21 has four pilot strips 17 in the embodiment 1, and the four pilot strips 17 form the pilot structure. And the pilot structure on the end cap 21 can also be changed to two pilot slices 18 (see Fig. 14) or one pilot tube 19 (see Fig. 15). It will also be understood and recognized by those skilled in the art.
10. In the above embodiment 1, the shoulder 13 can be omitted and it will also can make the trigger 4 head in the flush or retracted state relative to the shell 1 head when the lancet is in the post firing state. As shown in Fig. 16 and Fig. 17, Fig. 16 shows the section view of embodiment 1 of present invention in the initial assembly state omitting the shoulder and Fig. 17 shows the section view of embodiment 1 of present invention in the post firing state omitting the shoulder. As can be seen from Fig. 16, the shoulder 13 in the embodiment 1 is omitted, and the hook 12 on the trigger 4 cooperates with the inner end face 14 to form a first limit to prevent the forward movement of the trigger 4 relative to the shell 1. As can be seen from Fig. 17, the unlocking bevel 10 at the front of the pilot strip 17 presses against the middle of the elastic arm 5, and the end of the elastic arm 5 presses against the inner wall of the shell 1, so that the trigger 4 is in the clamped and positioned state in the shell 1, and in such state, the trigger 4 head is in the flush or retracted state relative to the shell 1 head and the trigger 4 can't return relative to the shell 1.
11. In the above embodiment 1, the spring 3 presses against the lancet core 2 at the front end, and if the lancet core 2 is sleeved into a component at the tail end or the separate lancet core is made, then the component presses against the spring, which will also be understood and recognized by those skilled in the art and falls within the protection scope of the present invention. The essence of the present invention herein is that the spring 3 acts on the core 2 at the front end, wherein there are two ways of direct action and indirect action. The way described above is the meaning of indirect action.

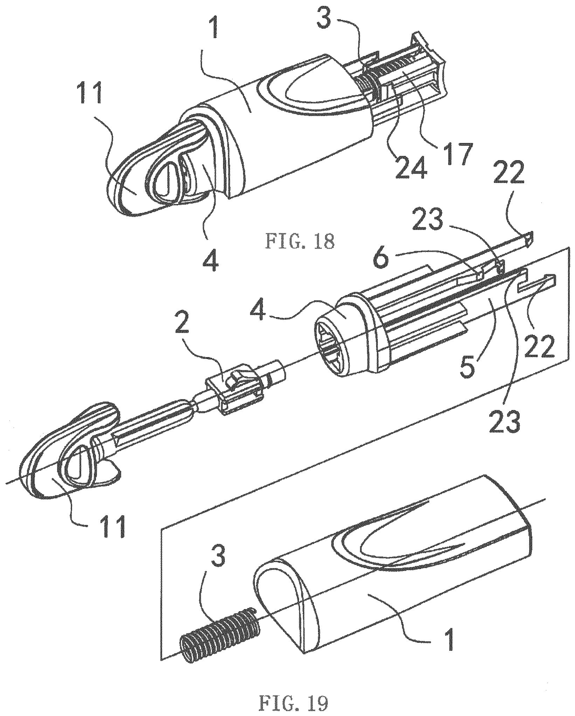

Embodiment 2: A pressing type disposable safety lancet with spring pilot structure (twist cap type, the limit structure between the trigger and the shell is different from the embodiment 1) As shown in Fig. 18-22, the disposal safety lancet consists of a shell 1, a twist cap 11, a lancet core 2, a spring 3 and a trigger 4 (see Fig. 18 and 19).

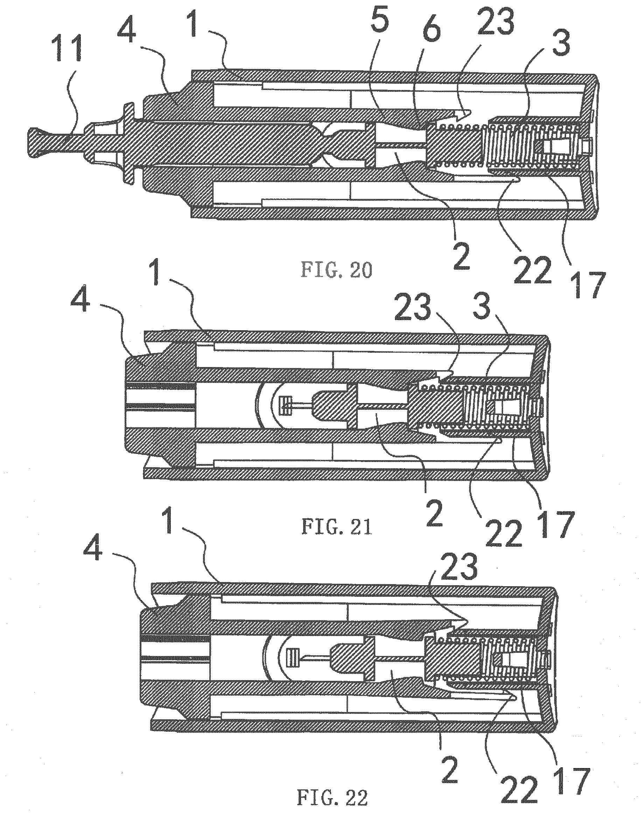

The difference between the embodiment 2 and the embodiment 1 is that: the embodiment 2 provides the cooperation structure of an initial hook 22 and a triggering hook 23 with the hook face 24 to replace the cooperation structure of the hook 12 and the shoulder 13 with the inner end face 14 in the embodiment 1. The specific structure of the embodiment 2 is: the trigger 4 is provided with an initial hook 22 and a triggering hook 23 at its tail (see Fig. 19), and the pilot structure is provided with a hook face 24 relative to the initial hook 22 and the triggering hook 23 (see Fig. 18). In the assembly state before firing, the initial hook 22 on the trigger 4 cooperates with the hook face 24 to form a first limit (see Fig. 20) to prevent the forward movement of the trigger 4 relative to the shell 1. In the post firing state, the triggering hook 23 on the trigger 4 cooperates with the hook face 24 to form a second limit (see Fig. 21 and Fig. 22) to prevent the trigger 4 from returning relative to the shell 1, so that the trigger 4 head is in the flush or retracted state relative to the shell 1 head.

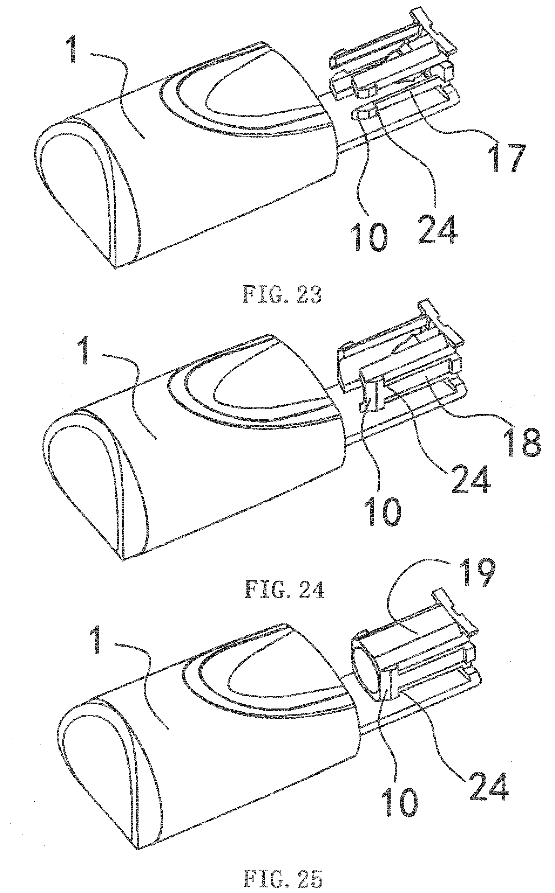

Fig. 23, Fig. 24 and Fig. 25 respectively shows the pilot structures with three different forms in the embodiment 2, wherein, the pilot structure shown in Fig. 23 is four pilot strips 17 and the hook face 24 is provided at the outside of the pilot strips 17; the pilot structure shown in Fig. 24 is two pilot slices 18 and the hook face 24 is provided at the outside of the pilot slice 18; the pilot structure shown in Fig. 25 is a pilot tube 19 and the hook face 24 is provided at the outside of the pilot tube 19.

The other contents are the same as those in the embodiment 1 and are not described repeatedly.

All the above possible variants related to the embodiment 1 are applicable to the embodiment 2, and are not described repeatedly.

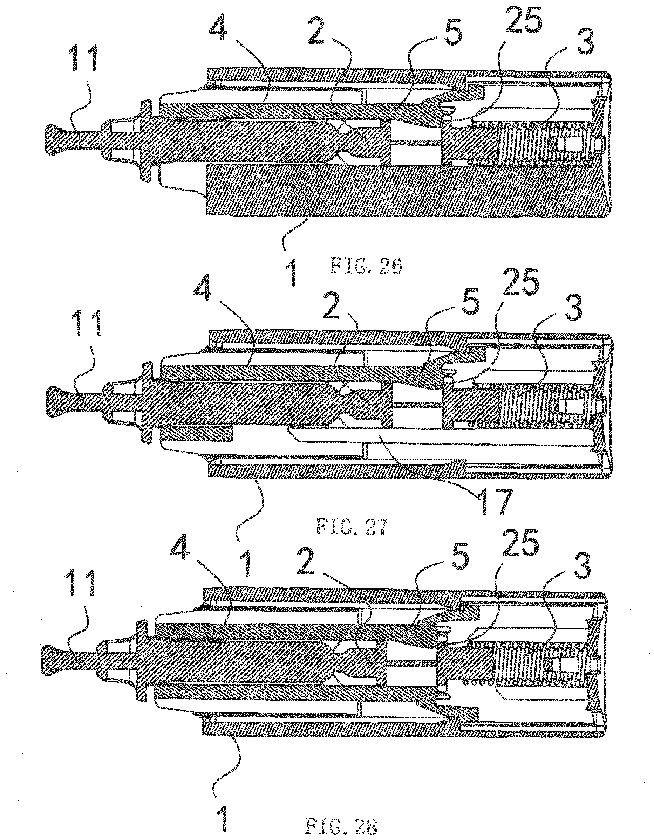

Embodiment 3: A pressing type disposable safety lancet with spring pilot structure (twist cap type, the locking mechanism between the trigger and the lancet core is different from the embodiment 1)
As shown in Fig. 26, the disposal safety lancet consists of a shell 1, a twist cap 11, a lancet core 2, a spring 3 and a trigger 4.

The difference between the embodiment 3 and the embodiment 1 is that: the locking mechanism between the trigger 4 and the lancet core 2 is different. The embodiment 1 is: the trigger 4 is provided with two elastic arms 5 at the tail (see Fig. 5), the elastic arm 5 is provided with a hook 6 to lock the lancet core 2, and the lancet core 2 is provided with a clamping face 8 relative to the hook 6 (see Fig. 3 and Fig. 4), two elastic arms 5 form an elastic clamp together, and the lancet core 2 is clamped, positioned and locked in the elastic clamp, and the clamping face 8 cooperates with the hook 6 to lock the lancet core 2 (see Fig. 8), thereby forming the locking mechanism. The embodiment 3 is: As shown in Fig. 26, the trigger 4 is provided with an elastic arms 5 at the tail, the elastic arm 5 is provided with an end face 25 to lock the lancet core 2, and the elastic arm 5 cooperates with the inner wall of the shell 1 to form an elastic clamp, and the lancet core 2 is clamped, positioned and locked in the elastic clamp, thereby forming the locking mechanism. It can be seen that the elastic clamp can be formed not only by at least two elastic arms, but also by the cooperation of at least one elastic arm 5 and the inner wall of the shell 1.

The other contents are the same as those in the embodiment 1 and are not described repeatedly.

All the above possible variants related to the embodiment 1 are applicable to the embodiment 3, and are not described repeatedly.

Embodiment 4: A pressing type disposable safety lancet with spring pilot structure (twist cap type, the locking mechanism between the trigger and the lancet core is different from the embodiment 1)
As shown in Fig. 27, the disposal safety lancet consists of a shell 1, a twist cap 11, a lancet core 2, a spring 3 and a trigger 4.

The difference between the embodiment 4 and the embodiment 1 is that: the locking mechanism between the trigger 4 and the lancet core 2 is different. The embodiment 4 is: As shown in Fig. 27, the trigger 4 is provided with an elastic arms 5 at the tail, the elastic arm 5 is provided with an end face 25 to lock the lancet core 2, and the elastic arm 5 cooperates with the inner wall of the pilot strip 17 to form an elastic clamp, and the lancet core 2 is clamped, positioned and locked in the elastic clamp, thereby forming the locking mechanism. It can be seen that the elastic clamp can be formed not only by at least two elastic arms, but also by the cooperation of at least one elastic arm 5 and the inner wall of the pilot strip 17.

The other contents are the same as those in the embodiment 1 and are not described repeatedly.

All the above possible variants related to the embodiment 1 are applicable to the embodiment 4, and are not described repeatedly.

Embodiment 5: A pressing type disposable safety lancet with spring pilot structure (twist cap type, the locking mechanism between the trigger and the lancet core is different from the embodiment 1)
As shown in Fig. 28, the disposal safety lancet consists of a shell 1, a twist cap 11, a lancet core 2, a spring 3 and a trigger 4.

The difference between the embodiment 5 and the embodiment 1 is that: the locking mechanism between the trigger 4 and the lancet core 2 is different. The embodiment 5 is: As shown in Fig. 28, the trigger 4 is provided with an elastic arms 5 at the tail, the elastic arm 5 is provided with an end face 25 to lock the lancet core 2, and the elastic arm 5 cooperates with the inner wall of the trigger 4 to form an elastic clamp, and the lancet core 2 is clamped, positioned and locked in the elastic clamp, thereby forming the locking mechanism. It can be seen that the elastic clamp can be formed not only by at least two elastic arms, but also by the cooperation of at least one elastic arm 5 and the inner wall of the trigger 4.

The other contents are the same as those in the embodiment 1 and are not described repeatedly.

All the above possible variants related to the embodiment 1 are applicable to the embodiment 5, and are not described repeatedly.

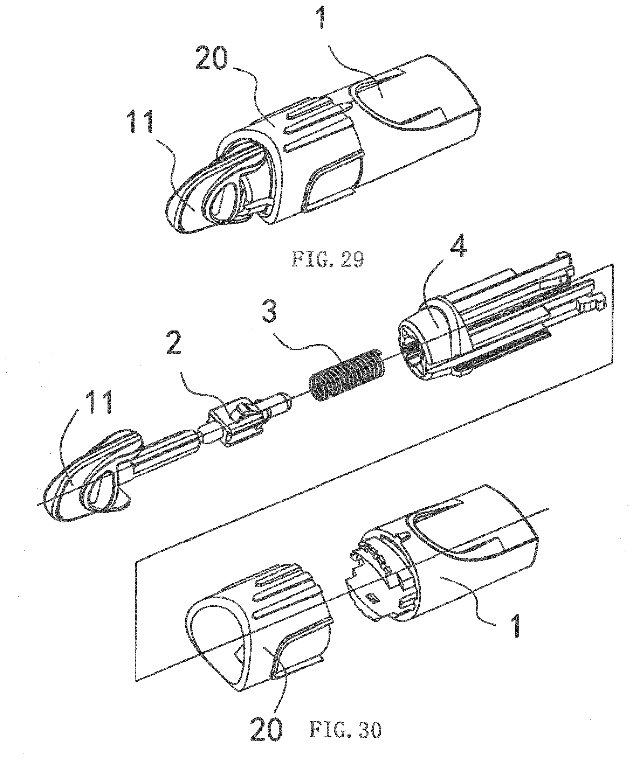

Embodiment 6: A pressing type disposable safety lancet with spring pilot structure (with a built-in adjusting head and twist cap type)
As shown in Fig. 29 and Fig. 30, the disposal safety lancet consists of a shell 1, an adjusting head 20, a twist cap 11, a lancet core 2, a spring 3 and a trigger 4.

The difference between the embodiment 6 and the embodiment 1 is that: the shell 1 head is provided with an adjusting head 20 structure which can change the puncture depth. The prior art can be used for the adjusting head 20 which can change the puncture depth, such as the structure disclosed in the Chinese Patent Application with the application number of 201610158481.8, the publication number of CN105816188A and the title of "An Improved Disposal Puncture Depth Adjustable Safety Lancet" or the structure disclosed in the Chinese Patent with the patent number of 201620212700.1, the authorization announcement number of CN205586011U and the title of "An Improved Disposal Puncture Depth Adjustable Safety Lancet". It will not be described in detail here.

The other contents are the same as those in the embodiment 1 and are not described repeatedly.

All the above possible variants related to the embodiment 1 are applicable to the embodiment 6, and are not described repeatedly.

It should be noted that the above described embodiments are only for illustration of technical concept and characteristics of present invention with purpose of making those skilled in the art understand the present invention, and thus these embodiments shall not limit the protection range of present invention. The equivalent changes or modifications according to spiritual essence of present invention shall fall in the protection scope of present invention.

## Claims

1. A pressing type disposable safety lancet with spring pilot structure comprising a shell (1), a lancet core (2), a spring (3) and a trigger (4);
The shell (1) forms a ejecting cavity, the lancet core (2) is positioned and locked in the ejecting cavity by the locking mechanism, the spring (3) acts on the lancet core (2) with its front end and a ejection guiding structure is provided between the lancet core (2) and the shell (1) or the trigger (4); The head of the trigger (4) extends out of the front end of the shell (1) and acts as the triggering end for blood sampling, the body of the trigger (4) is sliding connected in the shell (1) and the tail of the trigger (4) cooperates with the locking mechanism to form the unlocking mechanism, thereby forming the head pressing type firing structure;
Wherein: As for the spring (3) is provided with a pilot structure for piloting, the pilot structure is located in the shell (1) and arranged on the outer circumference of the spring (3); the pilot structure comprises at least three pilot strips (17), or at least two pilot slices (18), or a pilot tube (19), and the pilot tube (19) or all the pilot strips (17) or all the pilot slices (18) are arranged on the outer circumference of the spring (3) to form a pilot channel confining the spring (3), and the spring (3) is located in the pilot channel, so that the elastic force of the spring (3) acts on the ejection direction of the lancet core (2), and the rear end of the spring (3) acts on the pilot structure or the end cover of the shell (1); The locking mechanism is formed by the cooperation of the trigger (4) and the lancet core (2), wherein the trigger (4) is provided with at least an elastic arm (5) at the tail end and the elastic arm (5) is provided with a hook (6) or an end face (25) to lock the lancet core (2), and all the elastic arms (5) form an elastic clamp together or the elastic arm (5) forms an elastic clamp with one of the inner wall of the shell (1), inner wall of the trigger (4) and inner wall of the pilot structure, and the lancet core (2) is clamped, positioned and locked in the elastic clamp to form the locking mechanism;
The unlocking mechanism is formed by the cooperation of the pilot structure and the trigger (4), wherein, the front of the pilot structure is set to face the back of the elastic arm (5), and at least one of the front of the pilot structure and the back of the elastic arm (5) is set with an unlocking bevel (10) ;
Under the use state, when the trigger (4) head is pressed, the trigger (4) moves backwards in the shell (1), the front of the pilot structure hits the back of the elastic arm (5) and forces the end of the elastic arm (5) to open under the action of the unlocking bevel (10), and the lancet core (2) is fired for puncturing under the action of the spring (3) after the hook (6) or the end face (25) on the elastic arm (5) unhooks from the locking critical point.

2. The lancet according to claim 1, wherein: the pilot structure is the structure extending from the end cap or/and the inner wall of the shell (1).

3. The lancet according to claim 1, wherein: the trigger (4) is provided with an initial hook (22) and a triggering hook (23) at its tail, and the pilot structure is provided with a hook face (24) relative to the initial hook (22) and the triggering hook (23), and in the assembly state before firing, the initial hook (22) on the trigger (4) cooperates with the hook face (24) to form a first limit to prevent the forward movement of the trigger (4) relative to the shell (1); in the post firing state, the triggering hook (23) on the trigger (4) cooperates with the hook face (24) to form a second limit to prevent the trigger (4) from returning relative to the shell (1), so that the trigger (4) head is in the flush or retracted state relative to the shell (1) head.

4. The lancet according to claim 1, wherein: the trigger (4) is provided with a hook (12) and a shoulder (13) at the side and the shell (1) is provided with an inner end face (14) on the inner wall relative to the hook (12) and the shoulder (13) ; In the assembly state before firing, the hook (12) on the trigger (4) cooperates with the inner end face (14) to form a first limit to prevent the forward movement of the trigger (4) relative to the shell (1); in the post firing state, the shoulder (23) on the trigger (4) cooperates with the inner end face (14) to form a second limit to prevent the trigger (4) from returning relative to the shell (1), so that the trigger (4) head is in the flush or retracted state relative to the shell (1) head.

5. The disposable lancing device of claim 1, wherein: the lancet core (2) is provided with a twist cap (11) in the front to form a twist cap type head pressing type disposable lancet.

6. The disposable lancing device of claim 5, wherein: The twist cap (11) is positioned in the front of the lancet core (2) and it's connected with the lancet core (2) to be an injection molding structure, and the twist cap (11) is formed by the fixed connection of a twisting part (15) and a protection rod (16), and a retractile neck (7) that can be twisted off is provided between the protection rod (16) and the lancet core (2).

7. The disposable lancing device of claim 6, wherein: The trigger (4) is provided with a needle outlet hole (9) at its head part and the maximum diameter of the twisting part (15) is larger than the maximum diameter of the needle outlet hole (9), and the protection rod (16) and the lancet core (2) can be inserted through for assembly from the front end of the needle outlet hole (9).

8. The disposable lancing device of claim 1, wherein: the lancet core (2) is provided with a cover cap in the front to form a twist cap type head pushing type disposable lancet.

9. The disposable lancing device of claim 1, wherein: the shell (1) head is provided with an adjusting head (20) structure which can change the puncture depth.
